# EUROPEAN PATENT SPECIFICATION

(11) **EP 2 022 406 B1**
(45) Date of publication and mention of the grant of the patent: **20.10.2010**
(21) Application number: 08252518.9
(22) Date of filing: 24.07.2008
(51) Int. Cl.: A61B 10/02

(54) **Trigger fired radial plate specimen retrieval biopsy instrument**
Mit Auslöser betriebenes Biopsie-Instrument zur Probenentnahme mit Radialplatte
Instrument de biopsie de prélèvement de spécimen sur une plaque radiale à déclencheur

(30) Priority: 25.07.2007 US 782963
(43) Date of publication of application: 11.02.2009
(73) Proprietor: Ethicon Endo-Surgery, Inc., Cincinnati, OH 45242 (US)
(72) Inventor: Garrison, William A., Springdale, Ohio 45246 (US)
(74) Representative: Tunstall, Christopher Stephen

(56) References cited:
- EP-A- 1 932 482
- WO-A-2007/112751
- US-A- 5 526 822
- US-A- 6 083 177
- US-A1- 2007 239 067
- US-B1- 6 485 436

## Description

### BACKGROUND

Embodiments of the present invention relate in general to biopsy devices, and more particularly to biopsy devices having a cutter for severing tissue, and even more particularly to biopsy devices for multiple sampling with a probe remaining inserted.

During a core needle biopsy, a small tissue sample may be removed, allowing for a pathological assessment of the tissue, including an assessment of the progression of any cancerous cells that are found. The following patent documents disclose various core biopsy devices: U.S. Pat. No. 6,273,862, issued Aug. 14, 2001; U.S. Pat. No. 6,231,522, issued May 15, 2001; U.S. Pat. No. 6,228,055, issued May 8, 2001; U.S. Pat. No. 6,120,462, issued September 19, 2000; U.S. Pat. No. 6,086,544, issued July 11, 2000; U.S. Pat. No. 6,077,230, issued June 20, 2000; U.S. Pat. No. 6,017,316, issued Jan. 25, 2000; U.S. Pat. No. 6,007,497, issued Dec. 28, 1999; U. S. Pat. No. 5,980,469, issued Nov. 9, 1999; U.S. Pat. No. 5,964,716, issued Oct. 12, 1999; U.S. Pat. No. 5,928,164, issued July 27, 1999; U.S. Pat. No. 5,775,333, issued July 7, 1998; U.S. Pat. No. 5,769,086, issued June 23, 1998; U.S. Pat. No. 5,649,547, issued July 22, 1997; U.S. Pat. No, 5,526,822, issued June 18, 1996; and U.S. Pub. No. 2003/0199753, published Oct. 23, 2003 to Hibner et al.

Some biopsy devices may be regarded as "long stroke" or "short stroke." For instance, several "short stroke" biopsy devices are described in the following published patent applications: U.S. Pub. No. 2005/0215921, U.S. Pub. No. 2004/0153003. In some embodiments, the cutter is cycled across the side aperture, reducing the sample time. Several alternative specimen collection mechanisms are described that draw samples through the cutter tube, which may allow for taking multiple samples without removing the probe from the breast.

In U.S. Pub. No. 2006/0074345, tissue samples are drawn by vacuum proximally through the cutter tube into a serial tissue stacking assembly that preserves the order of sample taking, that can be visually observed through a transparent lumen, and that serves as a transport container to take for a pathology examination. Yet another biopsy device is disclosed in EP 1 932 481 A1.

Additional sample storage devices are disclosed in US 2008/0221480.

While a variety of tissue storage devices has been made and used, it is believed that no one prior to the inventor has made or used a device as recited in the present claims.

US 6 485 436 discloses a biopsy needle assembly for removal of multiple biopsy cores from a single needle penetration. The needle apparatus comprises an elongate assembly of paired sleeves with an open notch in the wall of the outer sleeve for engaging a tissue volume in the bore of that sleeve. An inner sleeve or blade member with a sharp blade edge is moveable from a retracted position to an extended position to excise the tissue volume, and to function as valve means to alter the open notch between an open position and a closed position. A looped inflow-outflow passageway system for using high-pressure fluid flows is provided to push or expel the excised tissue from the bore in the working end where the excised tissue is captured. The looped passageway system is coupled to a remote pressurization source.

### SUMMARY OF THE INVENTION

The invention provides a biopsy device according to claim 1. Optional features are included in the dependent claims.

### BRIEF DESCRIPTION OF THE DRAWINGS

While the specification concludes with claims particularly pointing out and distinctly claiming the present invention, it is believed the same will be better understood by reference to the following description, taken in conjunction with the accompanying drawings in which:
FIGURE 1 is a perspective view from a right side of a first version of a biopsy device with a sample storage platter.
FIGURE 2 is a perspective view from a left side of the biopsy device of FIG. 1 with a handle housing removed.
FIGURE 3 is an exploded view from an upper right-hand side vantage point of the sample storage platter and handle housing of the biopsy device of FIG. 1.
FIGURE 4 is an exploded view from a lower right-hand side vantage point of sample storage platter of FIG. 3.
FIGURE 5 is an exploded view of biopsy device of FIG. 1 with the handle housing and sample storage platter omitted.
FIGURE 6 is a detail view of the firing mechanism of the assembled biopsy device of FIG. 2 with omitted handle housing.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

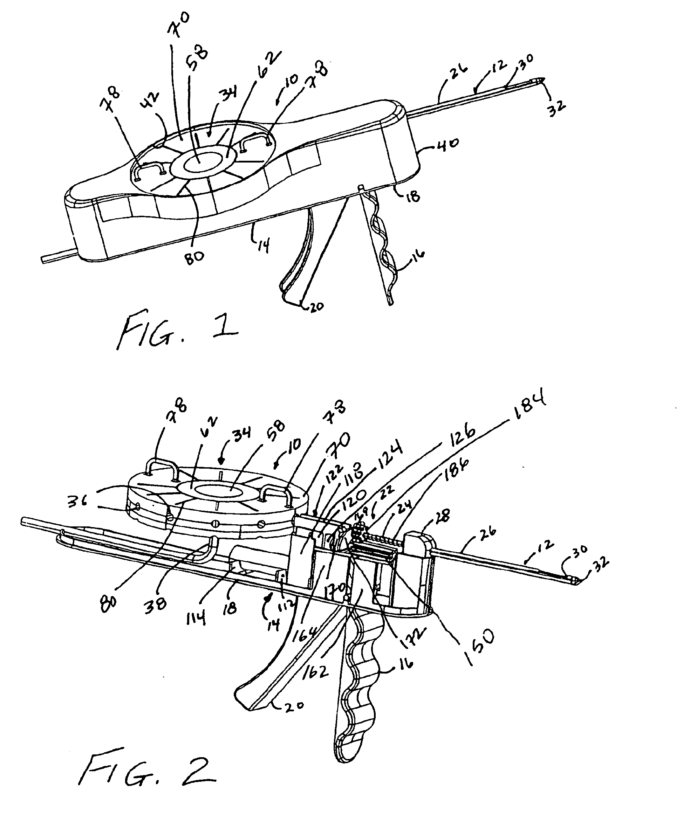

Turning to the drawings, wherein like numerals denote like components throughout the several views, in FIGS. 1-2, an exemplary biopsy device 10 has a biopsy needle (probe) 12 extending distally from a manually positioned and actuated handle 14. With particular reference to FIG. 2, a front trigger 16 downwardly extends pivotally from a handle base 18. A rear handle 20 extends from the handle base 18 aft of the front trigger 16. Actuation of front trigger 16 operates a firing mechanism 22 supported by the handle base 18 that longitudinally translates and rotates a hollow cutter 24 within a probe cannula 26 whose proximal end is attached to a probe mount 28 integral to the handle base 18. The cutter 24 translates across a side aperture 30 near a distal piercing tip 32 of the probe cannula 26. A sample storage platter 34 presents a plurality of radial sample cavities 36 that communicate with a central axial vacuum conduit 38 that is externally attached to a vacuum pump or institutional pneumatic supply (not shown). In particular, during an exemplary use of biopsy device 10, a vacuum draws tissue samples into cavities 36 as described in greater detail below. In FIG. 1, a handle housing 40 mates to the handle base 18 to encompass the firing mechanism 22 and to support for rotation the sample storage platter 34. Of course, these components are merely exemplary, and a variety of alternative components may be used in any other suitable combination. It will also be appreciated that some embodiments of a biopsy device 10 may be operable by a single hand of a user to obtain a biopsy sample.

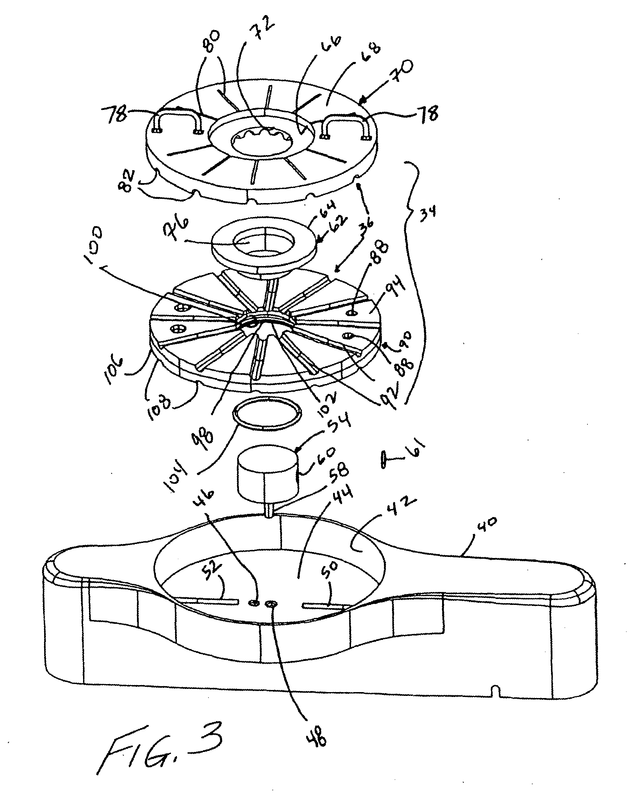

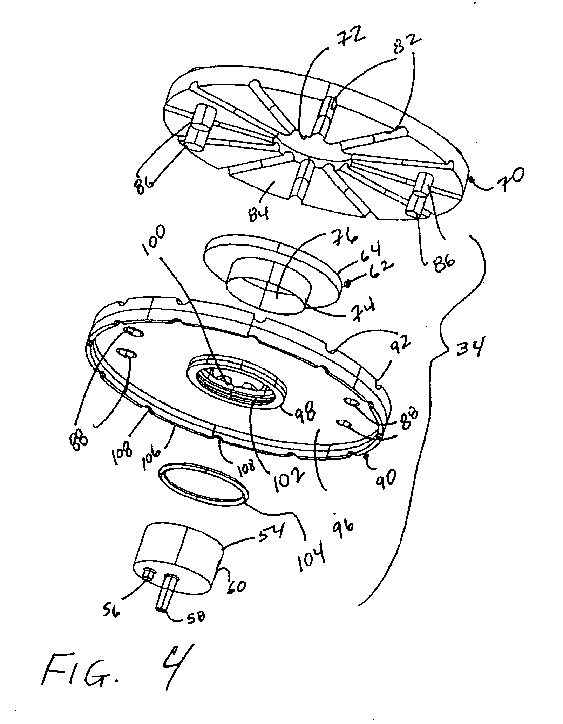

As shown in FIGS. 3-4, the handle housing 40 of the present example includes an upwardly open, cylindrical recess 42 shaped to closely receive the inserted sample storage platter 34. A bottom surface 44 of the cylindrical recess 42 includes an anti-rotation hole 46 just aft of a central mounting hole 48. A distally extending radial rib 50 and a proximally extending radial rib 52 form a longitudinally aligned diagonal on the bottom surface 44 for serving as an alignment feature, relatively spaced so that a cylindrical axle 54 may be received therebetween with an anti-rotation post 56 extending down into the anti-rotation hole 46 of the handle housing 40. A central post 58 of the cylindrical axle 54 extends downwardly for being received by the central mounting hole 48 and serves as a hose nib for receiving a distal end of the axial vacuum conduit 38 from below the bottom surface 44 of the handle housing 40. The central post 58 pneumatically communicates with a front port 60 on the cylindrical axle 54. In addition, a screen (not shown), filter, or other structure may be provided within the front port 60, between the front port 60 and an adjacent cavity 36 (e.g., as described below with respect to cylindrical body 74, etc.), or elsewhere, to permit fluid (e.g., liquid, air, etc.) to be drawn through the port 60 while preventing tissue samples from being drawn into the port 60. Alternatively, a storage platter 34 may be incorporated into a biopsy device 10 in any other suitable fashion using any other suitable structures or techniques.

The sample storage platter 34 of the present example is assembled from a capped cylindrical bushing 62 whose top horizontal ring 64 resides within a top shallow annular recess 66 formed on a top surface 68 of a top disk 70 surrounding a central aperture 72 through which a cylindrical body 74 of the bushing 62 passes with an inner diameter 76 dynamically sealing for rotation to an upper portion of the cylindrical axle 54 adjacent to the front port 60. In some embodiments, a screen (not shown), filter, or other structure is provided within the cylindrical body 74 of the bushing 62 to permit fluid (e.g., liquid, air, etc.) to be drawn through the cylindrical body 74 while preventing tissue samples from being drawn into the cylindrical body 74. By way of example only, the cylindrical body 74 may itself be formed entirely of a screen. Other ways in which filtration may be provided will be apparent to those of ordinary skill in the art.

The top disk 70 has a pair of upwardly extending and radially aligned drawer pull style grips 78 on opposite sides of the central aperture 72 for manual rotation of the sample storage platter 34 and/or removal of the storage platter 34 from the biopsy device 10. It will be appreciated by those of ordinary skill in the art, though, that grips 78 are merely exemplary, and that grips 78 may be substituted or supplemented by any other suitable structure (like any other component described herein), or may be omitted altogether.

By way of example only, an alternative embodiment may provide mechanically automated rotation and/or ejection of platter 34. For instance, a mechanism (not shown) may be coupled with trigger 16 or some other component to cause platter 34 to rotate to present an empty cavity 36 to cutter 24 after a biopsy sample has been obtained. Such a mechanism may thus cause platter 34 to cycle the cavities 36 in an automatic fashion as each biopsy sample is obtained using biopsy device 10. Suitable mechanisms for accomplishing such cavity 36 cycling, and ways in which such mechanisms may be incorporated into a biopsy device 10, will be apparent to those of ordinary skill in the art. As another merely illustrative example, a transmission and clutch (neither shown) may be provided in communication with a motor 110, whereby each actuation the trigger 16 combined with a subsequent release of the trigger 16 causes the clutch to couple the motor 110 with the platter 34 via the transmission in a manner sufficient to transfer rotation from the motor 110 to the platter 34 in a manner sufficient to align the next empty holding cavity 36 with the cutter 24. Suitable gearing ratios and other methods for accomplishing such rotation of a platter 34 by a motor 110 will be apparent to those of ordinary skill in the art. Furthermore, one or more locking mechanisms (not shown) may be provided on or within biopsy device 10 to prevent inadvertent rotation of the platter 34 during biopsy procedures.

In the present example, a plurality of radially spaced indicating grooves 80 are formed on the top surface of the top disk 70 of platter 34 indicating the location of corresponding radial sample cavities 36, though any other suitable indication may be used, to the extent that any indication is used. A top half of each cavity 36 is defined by grooves 82 formed on an undersurface 84 of the top disk 70. A bottom half of each cavity 36 is formed as a groove 92 formed on an upper surface 94 of the bottom disk 90. An O-ring seal 61 (FIG. 3) serves as a dynamic seal for the cavities 36 formed by grooves 82, 92 of the top and bottom disks 70, 90, reducing leakage between the outer opening defined by each cavity 36 and the cylindrical recess 42 during use of biopsy device 10.

Rotation of the bottom disk 90 is communicated to the top disk 70 by two pairs of posts 86 extending downwardly from the undersurface 84 of the top disk 70 and received into corresponding attachment holes 88 formed in a bottom disk 90. A bottom surface 96 of the bottom disk 90 includes a downwardly projecting central cylindrical lip 98 about a central aperture 100 of the bottom disk 90. The inner diameter of the cylindrical lip 98 has an O-ring recess 102 that receives an O-ring 104 positioned thereby to seal about the circumference of the cylindrical axle 54 below the front port 60. An outer cylindrical lip 106 extends downwardly from the outer circumference of the bottom disk 90 and includes radially spaced bottom notches 108 that correspond to each groove 92 and interact when aligned with the radial ribs 50, 52 to serve as detents for tactile feedback of proper alignment of the front port 60 to a selected sample cavity 36. In other embodiments, a platter 34 is formed as a single piece rather than having a top disk 70 and bottom disk 90. Alternatively, a platter 34 may be constructed in any other suitable way using any other suitable components.

Various components of the sample storage platter 34 may be formed of transparent or translucent materials to allow verification of a sample received within each cavity 36. In addition, the materials may be selected for not interfering with various diagnostic imaging modalities. For example, omitting ferrous materials may avoid safety issues near a strong magnetic field of a magnetic resonance imaging (MRI) machine. In addition, materials may be chosen to avoid artifacts or blocking of an imaging modality (e.g., computer aided tomatography (CT), MRI, etc.) so that preliminary tests can be made to confirm calcifications have been sampled to confirm correlation to a targeted suspicious lesion. Other suitable materials and properties that may be used will be apparent to those of ordinary skill in the art.

It should be appreciated that a filter may partially obstruct an inner end of each radial sample cavity 36 to allow air and liquid to be drawn away through the vacuum conduit 38 while retaining a sample in the sample storage platter 34. The attachment of the top disk 70 to the bottom disk 90 may also be readily detachable for retrieval of each sample. Alternatively, a platter 34 may otherwise be configured or manipulated for removal of tissue samples therefrom.

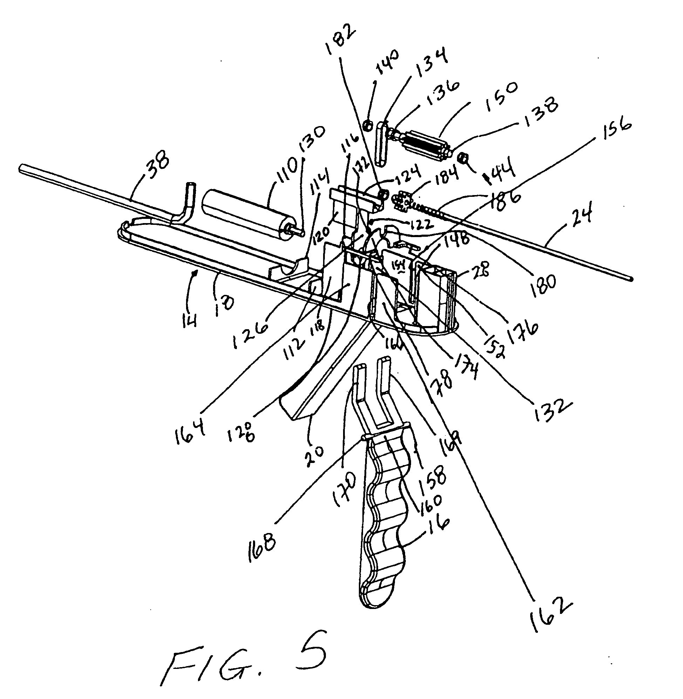

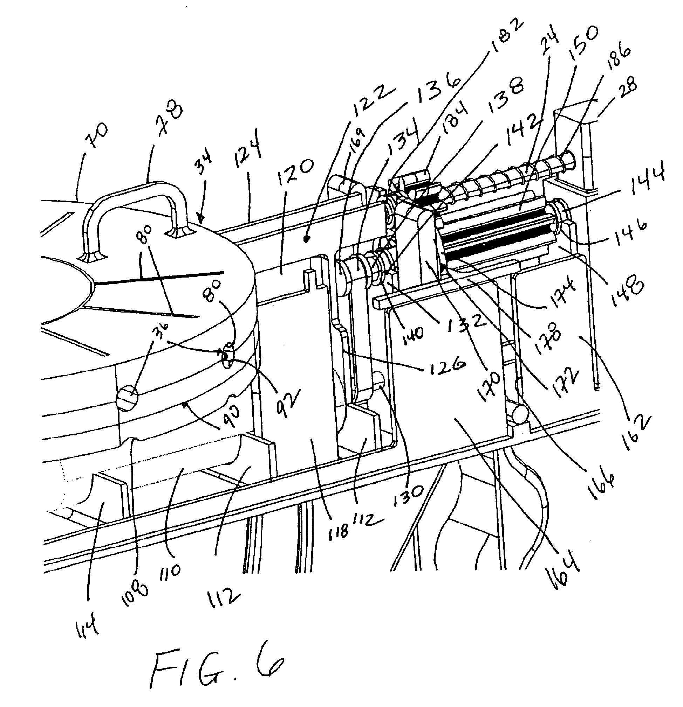

As shown in FIGS. 2, 5 and 6, the firing mechanism 22 of the present example employs the front trigger 16 for translation and a DC motor 110 with integral stored power for rotation of the cutter 24. Forward and aft transverse supports 112, 114 extend upwardly from the handle base 18 to support the DC motor 110. It will be appreciated, however, that the DC motor 110 is merely exemplary, and that a variety of alternative components may be used to rotate the cutter 24. For instance, any other type of motor (e.g., pneumatic, etc.) may be used. In other embodiments, cutter 24 does not rotate, such that the biopsy device 10 simply lacks a motor 110 or similar device altogether. It should also be understood that the term "rotate" may include a cutter 24 oscillating or rocking back and forth about an axis defined by the cutter 24. In other words, "rotation" need not be limited to one or more full rotations by a cutter 24 about an axis defined by the cutter 24.

Distal to the forward transverse support 112, longitudinally aligned left and right trough tabs 116, 118 extend upwardly to laterally receive and grip a narrowed rectangular portion 120 of a support structure 122 having a longitudinally aligned and upwardly open channel 124 along a top surface of the narrowed rectangular portion 120 that is upwardly closed by an undersurface of the handle housing 40 to form a sample conduit between a proximal opening of the cutter 24 and the selected radial sample cavity 36. Of course, a sample conduit between the cutter 24 and a selected radial cavity 36 may be provided in a variety of alternative ways by a variety of alternative structures, as will be appreciated by those of ordinary skill in the art.

The support structure 122 also includes a transverse, downwardly projecting tab 126, which is just distal to the narrowed rectangular portion 120, and which has a shaft hole 128 that supports a proximal exposed portion of a distally projecting drive shaft 130 of the DC motor 110. A transverse, upwardly projecting aft spur gear support 132 extends upwardly from the handle base 18 distal to and parallel with the downwardly projecting tab 126 to receive and support a distal end of the drive shaft 130. A transversely aligned drive belt 134 resides between the tab 126 and gear support 132, contacting a lower portion of the drive shaft 130 and going over a belt pulley 136 on a proximal end of a longitudinally aligned spur gear shaft 138. Distal to the belt pulley 136, a rivet shaped aft bearing 140 on the shaft 138 engages an upwardly open half cylinder recess 142 formed on top of the gear support 132. A front end of the shaft 138 includes a rivet-shaped front bearing 144 that engages an upwardly open half cylinder recess 146 formed on top of a front gear support 148. An elongate spur gear 150 resides between the aft and front gear supports 132, 148 for rotation by the shaft 138 centered therein. Activation of the DC motor 110 turns the drive belt 134 and thus the spur gear 150.

To the left of the spur gear 150, forward and aft left-side longitudinally aligned rectangular tabs 152, 154 extend upwardly from the housing base 18 and are spaced to define an upwardly open left trigger vertical slot 156 to receive a left pivot pin 158 extending laterally from a horizontal pivot axis 160 of the front trigger 16. To the right of the spur gear 150, forward and aft right-side longitudinally aligned rectangular tabs 160, 162 extend upwardly from the housing base 18 and are spaced to define an upwardly open right trigger vertical slot 166 to receive a right pivot pin 168 extending laterally from the front trigger 16. Left and right trigger fork tines 169, 170 extend upwardly from the horizontal pivot axis 160 of the front trigger 16, and are allowed to move longitudinally within respective cavities formed beneath the handle housing 40 between the spur gear 150 and respective pairs of tabs 152, 154 and tabs 162, 164.

A cutter shuttle 172 has a transverse, flat half ring 174 arching overtop of and spaced away from the spur gear 150 and attached on lateral sides to longitudinally aligned left and right elongate skids 176, 178 that slide on top respectively of the left tabs 152, 154 and right tabs 162, 164. An upwardly open rounded notch 180 formed at an apex of the half transverse flat ring 174 is engaged to a rivet-shaped cutter bearing 182 attached to a proximal end of the cutter 24. A follower spur gear 184 just distal to the cutter bearing 182 is also attached to the cutter 24 and is in gear engagement with the elongate spur gear 150 for rotation of the cutter 24. The cutter shuttle 172 may be urged distally by forward travel of the trigger tines 169, 170 as the forward trigger 16 is depressed aft. Upon release of the forward trigger 16, a compression spring 186 that resides on the cutter 24 between the probe mount 28 and the follower spur gear 184 provides a retracting force to the cutter 24. The cutter 24 maintains communication with the channel 24 during the range of translation.

In one merely exemplary use, a clinician depresses the trigger 16 of the biopsy device 10 to advance the cutter 24, closing the side aperture 30 in the probe 12, and inserts the probe 12 into tissue adjacent to a suspicious lesion, by gripping the rear handle 20, perhaps guided by ultrasonic imaging. Vacuum is provided through vacuum conduit 38 through the biopsy device 10 to side aperture 30 in the probe 12 to prolapse tissue into the side aperture 30. With the DC motor 110 activated, the cutter 24 rotates as the front trigger 16 is depressed, translating the cutter 24 across the side aperture 30, severing prolapsed tissue. Vacuum pressure draws the severed tissue sample into a distally aligned one of the radial sample cavities 36 in the sample storage platter 34. Then the probe 12 is repositioned if desired for an additional sample, and the grips 78 are manipulated to reposition an empty radial sample cavity 36 in the sample storage platter 34, and the process is repeated. Then the sample storage platter 34 may be lifted from the handle housing 40 for transport to pathology or on the spot imaging to verify appropriate samples. Although not shown, the packaging of individual platters 34 may serve as a fluid tight reusable package for transporting the used platter 34 to pathology. The samples may be extracted thereafter by pulling or drawing from the radial sample cavity 36 or by removal of the top disk 70 for direct access. Of course, these uses are merely exemplary, and those of ordinary skill in the art will appreciate that the biopsy device 10 of the present example and the numerous variations thereof may be used in a variety of alternative ways.

In some embodiments, the cutter 24 remains in constant rotation, independent of any actuation of the front trigger 16. For instance, a user actuated switch (not shown) may be provided on/in biopsy device 10, as a foot switch, or otherwise, for a user to activate the motor 110 to cause rotation of cutter 24. In other embodiments, a switch (not shown) may be operably coupled with the front trigger 16, such that a motor 110 begins rotating the cutter 24 as soon as the front trigger 16 is moved from a default position or is otherwise engaged or actuated. Alternatively, any other suitable structures, devices, or methods of activating a motor 110 may be used. In other embodiments, drive motor 110 is omitted altogether, and cutter 24 rotates using some other device or technique, or simply does not rotate at all.

While preferred embodiments of the present invention have been shown and described herein, it will be obvious to those skilled in the art, given the benefit of the present disclosure, that such embodiments are provided by way of example only. Numerous variations, changes, and substitutions will now occur to those skilled in the art without departing from the scope of the appended claims.

For example, a thumbwheel may included that allows rotation of the probe cannula 26 to orient the side aperture 30.

For another example, while manual translation of the cutter 24 provides certain advantages such as tactile feedback to the clinician, applications consistent with the present invention may include motorized translation or rotation that is converted from the translation motion of the cutter. Furthermore, hydraulic, pneumatic, or any other type of rotation and/or translation devices may be used.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and if necessary cleaned. The instrument may then be sterilized. In one sterilization technique, the instrument is placed in a closed an sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. the sealed container may keep the instrument sterile until it is opened in a medical facility. A device may also be sterilized using any other technique known in the art, including but not limited to beta or gamma radiation, ethylene oxide, or steam.

Having shown and described various embodiments of the present invention, further adaptations of the systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A biopsy device (10), comprising:
(a) a biopsy probe (12) having a distal opening (30);
(b) a cutter (24) translatable relative to the biopsy probe to sever tissue prolapsed into the distal opening of the biopsy probe, wherein the cutter defines a cutter longitudinal axis;
(c) a biopsy device body (40) attached to the biopsy probe comprising a pneumatic passage (38) communicating between a proximal end of the cutter and a vacuum source;
(d) a storage unit (34) movably received by the biopsy device body (40) to manually align a selected one of a plurality of tissue sample compartments (36) into communication with the pneumatic passage to receive severed tissue from the biopsy probe, wherein the storage unit comprises a plurality of tissue sample compartments arranged radially about a centre point, wherein the tissue sample compartments define elongate radial lumens extending outwardly in radial directions relative to the centre point, wherein the storage unit is rotatable about a rotation axis passing through the centre point, wherein the rotation axis is substantially perpendicular to the longitudinal axis defined by the cutter, wherein the storage unit is rotatable about the rotation axis to successively align each of the radial lumens with the longitudinal axis defined by the cutter to provide a path for a tissue sample communicated proximally through the cutter into an aligned radial lumen; and
(e) a transmission mechanism (22) operably configured to convert a trigger actuation motion into translation of the cutter tube; wherein the biopsy device is configured to be operable by a single hand of a user to obtain a biopsy sample.

2. The biopsy device (10) of claim 1, wherein the storage unit (34) comprises a platter, wherein the tissue sample compartments (36) comprise radial spoked sample compartments formed in the platter.

3. The biopsy device (10) of claim 2, wherein the platter comprises a disengageable disk (34) to expose the plurality of radial spoked sample compartments (36).

4. The biopsy device (10) of claim 1, wherein the storage unit (34) is manually rotatable by a user.

5. The biopsy device (10) of claim 6, wherein the storage unit (34) has at least one drawer pull grip (78) to facilitate manual rotation of the storage device.

6. The biopsy device (10) of claim 1, wherein the storage unit (34) has a substantially centrally located opening (58) for fluidly coupling a selected sample compartment (36) of the plurality of sample compartments with a vacuum source via the pneumatic passage (38).

7. The biopsy device (10) of claim 1, wherein the biopsy device body (40) comprises an opening (42) sized to allow replacement of the storage unit (34).

8. The biopsy device (10) of claim 1, wherein the distal opening (30) of the biopsy probe (12) comprises a side aperture.

9. The biopsy device (10) of claim 1, wherein the cutter (24) comprises a tube translatable within the biopsy probe.

10. The biopsy device (10) of claim 1, wherein the transmission mechanism (22) further comprises a rotation gear mechanism operably configured to impart a rotation to the cutter (24) during translation.

11. The biopsy device (10) of claim 1, wherein the biopsy probe comprises a cutter lumen and a vacuum lumen, wherein the cutter lumen is configured to receive the cutter (24), wherein the vacuum lumen is separate from but in fluid communication with the cutter lumen.

12. The biopsy device of claim 1, comprising:
a probe cannula (26) having a distal end (32) and the distal opening (30), wherein the cutter (24) is substantially hollow and is translatable within the probe cannula;
wherein the biopsy device body (40) is coupled with the probe cannula, and has a feature configured to be coupled with the vacuum source;
wherein the storage unit (34) has a plurality of tissue sample (36) compartments;
a mechanism operable to rotate the cutter within the probe cannula; and
a mechanism operable to translate the cutter within the probe cannula.

## Patentansprüche

1. Biopsiegerät (10) umfassend:
(a) eine Biopsiesonde (12) mit einer distalen Öffnung (30);
(b) eine Schneidvorrichtung (24), die relativ zur Biopsiesonde verschiebbar ist, um Gewebe abzutrennen, das in die distale Öffnung der Biopsiesonde vorgefallen oder prolabiert ist, wobei die Schneidvorrichtung eine Schneidvorrichtungslängsachse definiert;
(c) einen Biopsiegerätekörper (40) der an die Biopsiesonde angebracht ist, die einen pneumatischen Durchgang (38) umfasst, der eine Verbindung zwischen einem proximalen Ende der Schneidvorrichtung und einer Unterdruckquelle bereitstellt;
(d) eine Speichereinheit (34), die beweglich von dem Biopsiegerätekörper (40) aufgenommen ist, um ein ausgewähltes von mehreren Gewebeprobenabteilen (36) mit dem pneumatischen Durchgang ausrichtend zu verbinden, damit abgetrenntes Gewebe von der Biopsiesonde aufgenommen ist, wobei die Speichereinheit mehrere Gewebeprobenabteile umfasst, die radial um einen Mittelpunkt angeordnet sind, wobei die Gewebeprobenabteile längliche radiale Lumen definieren, die sich bezogen auf den Mittelpunkt in radiale Richtungen nach außen wegerstrecken, wobei die Speichereinheit um eine Drehachse drehbar ist, die durch den Mittelpunkt verläuft, wobei die Drehachse im Wesentlichen senkrecht zu der Längsachse steht, die durch die Schneidvorrichtung definiert ist, wobei die Speichereinheit um die Drehachse drehbar ist, um nacheinander jedes radiale Lumen mit der durch die Schneidvorrichtung festgelegten Längsachse auszurichten, um einen Pfad für eine Gewebeprobe bereitzustellen, die in proximaler Richtung durch die Schneidvorrichtung hindurch in ein ausgerichtetes radiales Lumen übertragen ist; und
(e) einen Übertragungsmechanismus (22), der betriebsgemäß dazu ausgelegt ist, eine Auslöserbetätigungsbewegung in eine Translationsbewegung des Schneidvorrichtungskanals umzuwandeln;
wobei das Biopsiegerät dazu ausgelegt ist, von einer einzelnen Hand einer Bedienperson bedient zu werden, um eine Biopsieprobe zu erhalten.

2. Biopsiegerät (10) nach Anspruch 1, wobei die Speichereinheit (34) eine Platte umfasst, wobei die Gewebeprobenabteile (36) radiale mit Speichen versehene Gewebeabteile umfassen, die in der Platte ausgebildet sind

3. Biopsiegerät (10) nach Anspruch 2, wobei die Platte eine entkuppelbare Scheibe (34) umfasst, um die mehreren radialen mit Speichen versehenen Probenfächer (36) freizulegen.

4. Biopsiegerät (10) nach Anspruch 1, wobei die Speichereinheit (34) von Hand durch eine Bedienperson drehbar ist.

5. Biopsiegerät (10) nach Anspruch 6, wobei die Speichereinheit (34) mindestens einen Anszuggriff (78) aufweist, um die manuelle Drehung der Speichereinheit zu erleichtern.

6. Biopsiegerät (10) nach Anspruch 1, wobei die Speichereinheit (34) eine im wesentlichen mittig platzierte Öffnung (58) aufweist zum fluidalen Verbinden eines ausgewählten Probenabteils (36) der mehreren Probenabteile mit einer Unterdruckquelle über einen pneumatischen Durchgang (38).

7. Biopsiegerät (10) nach Anspruch 1, wobei der Biopsiegerätekörper (40) eine Öffnung (42) aufweist, die derart bemessen ist, dass ein Ersetzen der Speichereinheit möglich ist.

8. Biopsiegerät (10) nach Anspruch 1, wobei die distale Öffnung (30) der Biopsiesonde (12) eine Seitenöffnung umfasst.

9. Biopsiegerät (10) nach Anspruch 1, wobei die Schneidvorrichtung (24) einen Kanal umfasst, der innerhalb der Biopsiesonde verschiebbar ist.

10. Biopsiegerät (10) nach Anspruch 1, wobei der Übertragungsmechanismus (22) einen Drehgatriebemechanismus aufweist, der betriebsgemäß dazu ausgelegt ist, der Schneidvorrichtung (24) während einer Translationsbewegung eine Drehbewegung mitzuteilen.

11. Biopsiegerät (10) nach Anspruch 1, wobei die Biopsiesonde ein Schneidvorrichtungslumen und ein Unterdrucklumen umfasst, wobei das Schneidvorrichtungslumen dazu ausgelegt ist, die Schneidvorrichtung (24) aufzunehmen, wobei das Unterdrucklumen von dem Schneidvorrichtungslumen getrennt, aber in fluidaler Kommunikation mit diesem ist.

12. Biopsiegerät nach Anspruch 1, umfassend:
eine Sondenkanüle (26) mit einem distalen Ende (32) und der distalen Öffnung (30), wobei die Schneidvorrichtung (24) im Wesentlichen hohl und verschiebbar innerhalb der Sondenkanüle ist;
wobei der Biopsiegerätekörper (40) mit der Sondenkanüle gekoppelt ist und eine Einrichtung aufweist, die dazu ausgelegt ist, mit der Unterdruckquelle verbunden zu sein;
wobei die Speichereinheit (34) mehrere Gewebeprobenabteile (36) aufweist;
einen Mechanismus zum Drehen der Schneidvorrichtung innerhalb der Sondenkanüle;
und einen Mechanismus zum Verschieben der Schneidvorrichtung innerhalb der Sondenkanüle.

## Revendications

1. Dispositif de biopsie (10), comprenant :
(a) une sonde de biopsie (12), ayant une ouverture distale (30) ;
(b) un découpeur (24) pouvant subir une translation relativement à la sonde de biopsie pour couper le tissu en procidence dans l'ouverture distale de la sonde de biopsie, dans lequel le découpeur définit un axe longitudinal de découpeur ;
(c) un corps de dispositif de biopsie (40) fixé à la sonde de biopsie comprenant un passage pneumatique (38) communiquant entre une extrémité proximale du découpeur et une source de vide ;
(d) une unité de stockage (34) reçue de manière mobile par le corps du dispositif de biopsie (40) afin d'aligner manuellement un élément choisi parmi une pluralité de compartiments d'échantillons de tissu (36) en communication avec le passage pneumatique, pour recevoir le tissu découpé de la sonde de biopsie, dans laquelle l'unité de stockage comprend une pluralité de compartiments d'échantillon de tissu disposés radialement autour d'un point central, dans laquelle les compartiments d'échantillon de tissu définissent des lumières radiales allongées s'étendant vers l'extérieur, dans des directions radiales relativement au point central, dans laquelle l'unité de stockage peut tourner autour d'un axe de rotation passant à travers le point central, dans laquelle l'axe de rotation est sensiblement perpendiculaire à l'axe longitudinal défini par le découpeur, dans laquelle l'unité de stockage peut tourner autour de l'axe de rotation, afin d'aligner successivement chacune des lumières radiales avec l'axe longitudinal défini par le découpeur, pour fournir un chemin pour un échantillon de tissu communiquant de manière proximale à travers le découpeur dans une lumière radiale alignée ; et
(e) un mécanisme de transmission (22) opérationnellement configuré pour convertir un mouvement d'actionnement de déclencheur en translation du tube de découpeur ; dans lequel le dispositif de biopsie est configuré pour pouvoir être actionné d'une seule main par un utilisateur, afin d'obtenir un échantillon de biopsie.

2. Dispositif de biopsie (10) selon la revendication 1, dans lequel l'unité de stockage (34) comprend un plateau, dans lequel les compartiments d'échantillon de tissu (36) comprennent des compartiments d'échantillon à rayons radiaux formés dans le plateau.

3. Dispositif de biopsie (10) selon la revendication 2, dans lequel le plateau comprend un disque débrayable (34) pour exposer la pluralité des compartiments d'échantillon à rayons radiaux (36).

4. Dispositif de biopsie (10) selon la revendication 1, dans lequel l'unité de stockage (34) peut être manuellement tournée par un utilisateur.

5. Dispositif de biopsie (10) selon la revendication 1, dans lequel l'unité de stockage (34) a au moins une poignée de tiroir (78) pour faciliter la rotation manuelle du dispositif de stockage.

6. Dispositif de biopsie (10) selon la revendication 1, dans lequel l'unité de stockage (34) a une ouverture située sensiblement au centre (58) permettant de raccorder de manière fluidique un compartiment d'échantillon choisi (36) parmi la pluralité de compartiments d'échantillons avec une source de vide, par le biais du passage pneumatique (38).

7. Dispositif de biopsie (10) selon la revendication 1, dans lequel le corps du dispositif de biopsie (40) comprend une ouverture (42) dimensionnée pour permettre le remplacement de l'unité de stockage (34).

8. Dispositif de biopsie (10) selon la revendication 1, dans lequel l'ouverture distale (30) de la sonde de biopsie (12) comprend une ouverture latérale.

9. Dispositif de biopsie (10) selon la revendication 1, dans lequel le découpeur (24) comprend un tube pouvant subir une translation dans la sonde de biopsie.

10. Dispositif de biopsie (10) selon la revendication 1, dans lequel le mécanisme de transmission (22) comprend en outre un mécanisme d'engrenage de rotation configuré de manière opérationnelle pour provoquer la rotation du découpeur (24) pendant la translation.

11. Dispositif de biopsie (10) selon la revendication 1, dans lequel la sonde de biopsie comprend une lumière de découpeur et une lumière de vide, dans lequel la lumière de découpeur est configurée pour recevoir le découpeur (24), dans lequel la lumière de vide est séparée mais en communication fluidique avec la lumière de découpeur.

12. Dispositif de biopsie selon la revendication 1, comprenant :
■ une canule de sonde (26) ayant une extrémité distale (32) et l'ouverture distale (30), dans laquelle le découpeur (24) est sensiblement creux et peut subir une translation dans la canule de sonde ;
■ dans lequel le corps du dispositif de biopsie (40) est raccordé à la canule de sonde, et a une caractéristique configurée pour être raccordée à la source de vide ;
■ dans lequel l'unité de stockage (34) a une pluralité de compartiments d'échantillons de tissu ;
■ un mécanisme actionnable pour tourner le découpeur dans la canule de sonde ; et
■ un mécanisme actionnable pour permettre la translation du découpeur dans la canule de sonde.
